# EUROPEAN PATENT SPECIFICATION

(11) **EP 2 620 773 B1**
(45) Date of publication and mention of the grant of the patent: **18.03.2015**
(21) Application number: 12152603.2
(22) Date of filing: 26.01.2012
(51) Int. Cl.: G01N 33/68, G01N 33/74

(54) **Method for the differentiation of forward and backward heart failure**
Verfahren zur Differenzierung eines Vor- und Rückwärtsherzversagens
Procédé de différentiation de l'insuffisance cardiaque antérograde et rétrograde

(43) Date of publication of application: 31.07.2013
(73) Proprietor: Roche Diagnostics GmbH, 68305 Mannheim (DE); F. Hoffmann-La Roche AG, 4070 Basel (CH)
(72) Inventor: Hess, Georg, 55130 Mainz (DE); Galluser, Andreas, 82377 Penzberg (DE); Horsch, Andrea, 6006 Luzern (CH); Klemt, Volker, 82362 Weilheim (DE); Zdunek, Dietmar, 82327 Tutzing (DE)
(74) Representative: Dick, Alexander

(56) References cited:
- EP-A1- 1 882 947
- US-A1- 2006 166 276
- DE PASQUALE CARMINE G ET AL: "Plasma surfactant protein-B - A novel biomarker in chronic heart failure", CIRCULATION, LIPPINCOTT WILLIAMS & WILKINS, US, vol. 110, no. 9, 31 August 2004 (2004-08-31), pages 1091-1096, XP002406007, ISSN: 0009-7322, DOI: 10.1161/01.CIR.0000140260.73611.FA
- D. MAGRI ET AL: "Circulating Plasma Surfactant Protein Type B as Biological Marker of Alveolar-Capillary Barrier Damage in Chronic Heart Failure", CIRCULATION: HEART FAILURE, vol. 2, no. 3, 30 March 2009 (2009-03-30), pages 175-180, XP55021392, ISSN: 1941-3289, DOI: 10.1161/CIRCHEARTFAILURE.108.819607
- DALZELL J R ET AL: "Novel biomarkers in heart failure: An overview", BIOMARKERS IN MEDICINE, FUTURE MEDICINE, LONDON, vol. 3, no. 5, 1 January 2009 (2009-01-01) , pages 453-463, XP009157303, ISSN: 1752-0363, DOI: 10.2217/BMM.09.42
- CLAUS LÜERS ET AL: "Importance of Surfactant Proteins B and D for the Differential Diagnosis of Acute Dyspnea", MEDIZINISCHE KLINIK, vol. 105, no. 9, 1 September 2010 (2010-09-01), pages 611-618, XP55021316, ISSN: 0723-5003, DOI: 10.1007/s00063-010-1100-0

## Description

The present invention relates to the field of laboratory diagnostics. Specifically, methods for differentiating in a heart failure patient (i) forward heart failure, (ii) backward heart failure of the left ventricle and (iii) forward heart failure and backward heart failure of the left ventricle are disclosed, based on the biomarkers proSP-B and/or NT-proBNP.

Heart failure (HF) represents a major public health problem in industrialized nations and appears to be the only common cardiovascular condition that is increasing in prevalence and incidence. In the United States, heart failure is responsible for almost 1 million hospital admissions and 40,000 deaths annually. The cardinal manifestations of heart failure are dyspnea and fatigue. Heart failure arises secondary to abnormalities of cardiac structure and/or function that impair the ability of the left ventricle to fill or eject blood. Said disease is now recognized as a systemic syndrome characterized by mal-adaptive neurohormonal, metabolic and inflammatory processes. The recognition of these pathways has prompted the evaluation of some of their components as biomarkers in both chronic heart failure and acutely decompensated heart failure.

For example, Lüers et al. (Med. Klin. 2010, 105, 611) have used the biomarkers SP-B, SP-D and NT-proBNP for differential diagnosis of acute dyspnea. It has been found in this study, that plasma levels of NT-proBNP were significantly higher in patients with a cardiac origin of acute dyspnea, in comparison to patients with a non-cardiac diagnosis. SP-D levels were highest in patients with a cardiac origin of acute dyspnea. However, after performing regression analysis, this finding appears to be of less importance for the differential diagnosis of acute dyspnea, as compared to NT-proBNP. In addition, the plasma levels of SP-B were found not different between the four subgroups analyzed in this study.

Magri et al. (Circ. Heart Fail. 2009, 2, 175) have described in another study that circulating SP-B values were increased in patients with heart failure. Patients suffering from most severe heart failure had the highest levels of SP-B. Furthermore, SP-B in the plasma has been regarded as a biological marker for alveolar-capillary barrier damage. It has also been reported that an increase of SP-B in patients with heart failure was due to an acute, though transient, increase in pulmonary microvascular pressure (Pmv) such as observed, e.g., during an acute cardiogenic edema. The increase in pulmonary microvascular pressure resulted in a mechanical disruption of the alveolar-capillary barrier with an increased leakage of SP-B into the bloodstream.

Guazzi et al. (Therapy 2005, 2, 641) have reported that elevated SP-B levels were associated with cardiogenic pulmonary edema.

WO 2004/077056 has described a method of diagnosing heart failure using the biomarkers NT-proBNP and SP-B or proSP-B and a corresponding method of monitoring heart failure development. Surfactant proteins, in particular surfactant protein B (SP-B) maintaining alveolar health, have been found to be associated with heart failure and NYHA classes. In addition, SP-B levels have been found to be raised in patients with chronic heart failure. Said levels fell, following treatment with intravenous diuretic.

To provide effective treatment for patients with heart failure, rapid and accurate differentiation of the causes and consequences of heart failure is necessary. However, said suitable means and methods are not yet available, due to the difficulty to identify the underlying major mechanisms.

Accordingly, the technical problem underlying the present invention can be seen as the provision of means and methods for complying with the aforementioned needs. The technical problem is solved by the embodiments characterized in the claims and herein below.

The present invention provides for differential diagnostic methods of forward heart failure and/or backward heart failure as consequences of heart failure, based on the determination of the biomarkers proSP-B (and/or the C-fragment proSP-B) and NT-proBNP. Thereby, said methods allow for an improved therapeutic guidance for physicians treating patients suffering from heart failure. In addition, the invention pertains to monitoring methods which allow to screen for modulations of blood levels of proSP-B (and/or the C-fragment proSP-B) and NT-proBNP in a heart failure patient. By this way, the monitoring methods of the invention can be used in order to follow forward heart failure and/or backward heart failure of the left ventricle in the heart failure patient, for example, during treatment.

More specifically, the present invention relates to method for differentiating in a heart failure patient between (i) forward heart failure, (ii) backward heart failure of the left ventricle and (iii) forward heart failure and backward heart failure of the left ventricle, the method comprising the steps of:
a) determining in a sample of a heart failure patient the ratio of the amount of proSP-B and the amount of NT-proBNP; and
b) comparing the ratio of the amount of proSP-B and the amount of NT-proBNP determined in step a) to at least one reference ratio of proSP-B and NT-proBNP,
thereby differentiating between (i) forward heart failure, (ii) backward heart failure of the left ventricle and (iii) forward heart failure and backward heart failure of the left ventricle in said heart failure patient.

In a preferred embodiment, the method for differentiating in a heart failure patient between (i) forward heart failure, (ii) backward heart failure of the left ventricle and (iii) forward heart failure and backward heart failure of the left ventricle, comprises the steps of:
a) determining in a sample of a heart failure patient the amount of proSP-B and the amount of NT-proBNP;
b) calculating the ratio of the amount of proSP-B and the amount of NT-proBNP; and
c) comparing the ratio of proSP-B and NT-proBNP determined in step b) to at least one reference ratio of proSP-B and NT-proBNP,
thereby differentiating between (i) forward heart failure, (ii) backward heart failure of the left ventricle and (iii) forward heart failure and backward heart failure of the left ventricle in said heart failure patient.

In another preferred embodiment of this method of the invention, the at least one reference ratio is derived (i) from a sample of a reference heart failure patient suffering from forward heart failure, (ii) from a sample of a reference heart failure patient suffering from backward heart failure of the left ventricle and/or (iii) from a sample of a reference heart failure patient suffering from forward heart failure and backward heart failure of the left ventricle. It is evident to those skilled in the art that the above mentioned reference ratio(s) can not only be derived from a single reference heart failure patient but also from a group or cohort of reference heart failure patients, as set forth elsewhere herein.

As demonstrated in the following Examples, a total of 92 patients with a median age of 72 years with overt heart failure have been included into the present study. Among these patients, the amounts of NT-proBNP as a biomarker for cardiac function and proSP-B as a biomarker for pulmonary damage have been analyzed in a serum sample. As shown in Figure 1 and Example 3, NT-proBNP amounts did not correlate with proSP-B amounts, confirming that both biomarkers provide for different information. ProSP-B levels correlated quite well to C-fragment proSP-B levels, except in a few patients who had high proSP-B and C-fragment proSP-B levels, as shown in Figure 2. In patients with cardiac decompensation, proSP-B amounts tended to increase with systolic pulmonary artery pressure which is considered to be a sign of backward heart failure. These results can be derived from Figure 3.

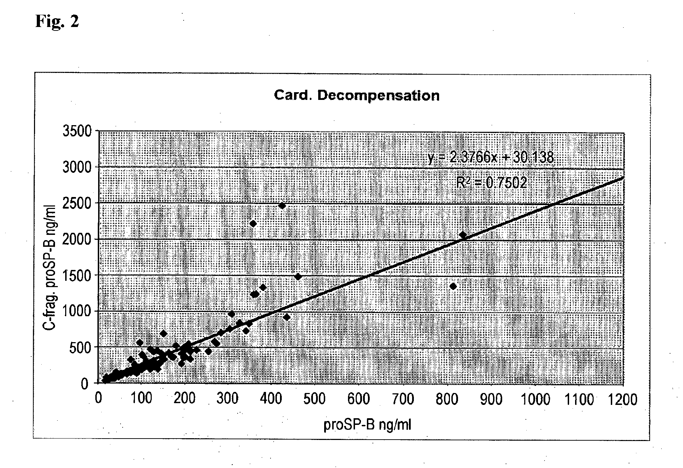

While NT-proBNP and proSP-B have been linked to heart failure and NYHA status in previous studies, no information has been provided so far as to the preferred consequence or manifestation of heart failure. By determining the amounts of proSP-B and NT-proBNP in samples of heart failure patients, calculating the ratios of proSP-B and NT-proBNP and comparing said ratios to reference ratios of proSP-B and NT-proBNP, respectively, it has advantageously been found by the present inventors that it is possible to differentiate between (i) forward heart failure, (ii) backward heart failure of the left ventricle and (iii) forward heart failure and backward heart failure of the left ventricle in heart failure patients. These results are demonstrated in the following Examples. Unexpectedly, it has further been found that the analysis of the amounts of proSP-B and C-fragment proSP-B in blood (serum) samples of heart failure patients provide largely identical information, indicating that the data obtained for said markers is comparable, as can be derived from Figure 2. Therefore, C-fragment proSP-B can be used instead of proSP-B or, in addition, to proSP-B in the means and methods of the invention. Moreover, it has been found in the present invention that increased NT-proBNP levels can be detected in said samples of heart failure patients suffering from forward heart failure but also in patients having backward heart failure of the left ventricle. NT-proBNP levels in patients having forward heart failure are comparable to those in patients with low output heart failure, whereas NT-proBNP levels in patients having backward heart failure of the left ventricle are comparable to those in patients with high output heart failure. High output heart failure can pass into low output heart failure (Kress et al., Harrison Principles of Internal Medicine, 17th edition, chapter 261, p. 1673 ff., Figure 261-2). Forward heart failure is associated with the inability of the heart to pump blood at a sufficient rate to meet the oxygen demands of the body. In this condition, the problem is primarily to supply adequate arterial perfusion in front of the heart preload. Accordingly, only a low output can be found in the aorta in patients with forward heart failure. As a further unexpected result, backward failure of the left ventricle is associated with an increased proSP-B level, most likely as a result of the congestion of the pulmonary vasculature which leads to leakage of the hydrophilic proSP-B from the Alveolar Type II cells into the circulation. In contrast, there is no or less congestion of the pulmonary vasculature reflected in non-elevated proSP-B levels, in patients suffering from backward heart failure of the right ventricle. Thus, increased proSP-B amount is a marker of backward failure of the left ventricle.

The data presented herein further suggest that a correlation exists in relation to the quantitative level of proSP-B (and/or C-fragment proSP-B) which is observed in the blood sample of a patient suffering from backward heart failure of the left ventricle and the extent of the involvement of the lung. Specifically, the higher the level of said surfactant, the more severe the involvement and damage of the alveolar type II cells of the lung caused by the increased venous pressure behind the heart afterload seems to be.

In light of these findings, the methods of the invention allow for a more precise treatment regimen of heart failure patients, in comparison to those described in the prior art. By differentially diagnosing forward heart failure and/or backward heart failure of the left ventricle in a patient suffering from heart failure, an efficient therapeutic guidance is provided for the treating physician by unraveling the consequences of heart failure. For example, forward heart failure is in general associated with low left ventricular output and peripheral ischemia. In this case, volume substitution can be indicated in order to avoid cardio-renal syndrome, whereas loop diuretics might not be useful from a therapeutic point of view. Furthermore, inotropes such as doputamine can be useful in this scenario. ASSIST devices can also be helpful to support output in cases that do not respond to pharmacotherapy. In contrast, in case of pulmonary congestion as is the case in backward heart failure, reduction of intravasal volume is the therapy of choice. This includes the use of loop diuretics or vasodilators such as nitrates. By allowing rapid and accurate differentiation of heart failure causes, the means and methods of the invention provide not only improved therapy of heart failure patients but also cost-effective treatment of said patients.

The term "differentiating" as used herein means distinguishing between (i) forward heart failure, (ii) backward heart failure of the left ventricle and (iii) forward heart failure and backward heart failure of the left ventricle as the consequence of an apparent heart failure in a subject. The term differentiating as referred to herein, preferably, includes differentially diagnosing each of the aforementioned condition. Differentiating as used herein refers to assessing the probability according to which a subject suffers from forward heart failure or backward heart failure of the left ventricle. As will be understood by those skilled in the art, such an assessment is usually not intended to be correct for 100% of the subjects to be diagnosed. The term, however, requires that a statistically significant portion of the subjects can be diagnosed to suffer from the said disease (e.g., a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Even more preferred, "differentiating" as referred to herein means distinguishing between (i) forward heart failure and (ii) backward heart failure of the left ventricle, as the consequence of an apparent heart failure in a subject.

The term "diagnosing" according to the present invention includes detecting, monitoring, confirmation, sub-classification and prediction of the relevant disease set forth herein, the symptoms or risks for it. "Monitoring" as used herein relates to keeping track of an already diagnosed disease, or complication, e.g. to analyzing the progression or consequence of the disease or the influence of a particular treatment on the progression of disease or complication. For example, said monitoring comprises screening for the modulation of blood levels of proSP-B and/or the C-fragment proSP-B and/or NT-proBNP in a heart failure patient in order to detect and/or follow forward heart failure and/or backward heart failure of the left ventricle. To this end, the amount or ratio of said biomarkers can be determined at various time points (TP or T) TP0/T0, TP1/T1, TP2/T2, TP3/T3 and so forth, for example, during heart failure therapy. The time period between the indicated time points is preferably about 3 hours, 4 hours, 5 hours, 6 hours, 12 hours, 24 hours, 2 days, 3 days, 4 days, 5 days, 6 days, 7 days, 10 days, two weeks, three weeks, four weeks, six weeks, 2 months, 3 months or even longer. It is evident to those skilled in the art that the amount or ratio of said biomarkers is determined not only at two, three or four time points but also at five, six, seven or even more time points. In addition, the time period between said time points can be constant or can vary. For example, the time period between TP0/T0, TP1/T1, TP2/T2, TP3/T3 and so forth can be always 2 weeks. Or the time period between TP1/T1 and TP2/T2 may be, e.g., about a few hours (for instance, about 3, 4, 5, 6, 12, or 24 hours) and the time period between TP2/T2 and TP3/T3 can be about 2, 3, 4, or 8 weeks. Said modulation of the blood levels of proSP-B and/or the C-fragment proSP-B and/or NT-proBNP can be, for example, an increase or decrease of the amount or ratio of the mentioned biomarkers. Preferably, the increase or decrease is a modulation of at least 10%, 15%, 20%, 25%, 30% or even more, in comparison to the amount or ratio of said biomarkers determined at a previous time point. For instance, a (statistically significant) increase of proSP-B and/or C-fragment proSP-B can be indicative of backward heart failure. If the amount of proSP-B and/or C-fragment proSP-B remains essentially stable, there is no evidence of backward heart failure. A significant/strong increase of NT-proBNP is rather indicative of a change of the cardiac function; see, e.g., Examples 4 and 5. Confirmation relates to the strengthening or substantiating a diagnosis already performed using other indicators or markers. Sub-classification relates to further defining a diagnosis according to different subclasses of the diagnosed disease, e.g. defining according to mild and severe forms of the disease. Prediction relates to the prognosis of a disease or complication before other symptoms or markers have become evident or have become significantly altered.

The term "subject" or "test subject" as used herein relates to animals, preferably mammals, and, more preferably, humans. However, it is envisaged by the present invention that the subject shall, preferably, exhibit the apparent clinical symptoms of a heart failure well known in the art. Preferably, the subject or test subject is a human heart failure patient. The cardinal manifestations of heart failure are dyspnea and fatigue. Heart failure may be also associated with abnormal ejection fraction as assessed by echocardiography or may have preserved ejection fraction (Hess and Carroll, Braunwald's heart disease 9th edition, Chapter 23, 561 ff.). In a preferred embodiment of the means and methods of the invention, the subject(s) (as well as the subject(s) from which the reference amount or reference ratio is (are) derived) as referred to herein does (do) not have impaired renal function. How to assess whether a subject exhibits impaired renal function is well known in the art. Renal disorders can be diagnosed by any means known and deemed appropriate. Particularly, renal function can be assessed by means of the glomerular filtration rate (GFR). For example, the GFR may be calculated by the Cockgroft-Gault or the MDRD formula (Levey 1999, Annals of Internal Medicine, 461-470). GFR is the volume of fluid filtered from the renal glomerular capillaries into the Bowman's capsule per unit time. Clinically, this is often used to determine renal function. The GFR was originally estimated (the GFR can never be determined, all calculations derived from formulas such as the Cockgroft Gault formula of the MDRD formula deliver only estimates and not the "real" GFR) by injecting inulin into the plasma. Since inulin is not reabsorbed by the kidney after glomerular filtration, its rate of excretion is directly proportional to the rate of filtration of water and solutes across the glomerular filter. In clinical practice, however, creatinine clearance is used to measure GFR. Creatinine is an endogenous molecule, synthesized in the body, which is freely filtered by the glomerulus (but also secreted by the renal tubules in very small amounts). Creatinine clearance (CrCl) is therefore a close approximation of the GFR. The GFR is typically recorded in milliliters per minute (mL/min). The normal range of GFR for males is 97 to 137 mL/min, the normal range of GFR for females is 88 to 128 ml/min. Thus, it is particularly contemplated that the GFR of a subject who does not exhibit impaired renal function is within this range. Moreover, said subject preferably, has a blood creatinine level (in particular a serum creatinine level) of lower than 0.9 mg/dl, more preferably of lower than 1.1 mg/dl and most preferably of lower than 1.3 mg/dl.

Preferably, human patients having renal insufficiency or from right sided heart failure are disclaimed or excluded from the means and methods of the invention. In another preferred embodiment of the means and methods of the invention, the subject as referred to herein does not suffer from a primary or secondary lung disease or disorder. Preferably the subject does not suffer from lung disorders which are known to the person skilled in the art and described, e.g., in Harrison Principles of Internal Medicine, Chapters 245 - 260, pp. 1583 ff (17th Edition). Specifically, pulmonary disorders include hypersensitivity pneumonitis, environmental lung disorders, such as asbestosis, silicosis, farmers lung, lung disorders caused by toxic chemic agents, such as tobacco smoke, formaldehyde and ozone, pneumonia, cystic fibrosis, chronic obstructive lung disease, asthma bronchiale, bronchoectasia and interstitial lung disorders, such as systemic lupus erythematodes, Morbus Wegener or Goodpasture syndrome and ARDS (acute respiratory distress syndrome) or its precursors, frequently associated with systemic infections, trauma or ventilation.

"Determining" the amount of a polypeptide referred to herein, e.g., a natriuretic peptide, such as NT-proBNP and/or a pulmonary surfactant protein, such as proSP-B or C-fragment proSP-B, relates to measuring the amount or concentration of said polypeptide, preferably semi-quantitatively or quantitatively. Measuring can be done directly or indirectly. Direct measuring relates to measuring the amount or concentration of the polypeptide based on a signal which is obtained from the polypeptide itself and the intensity of which directly correlates with the number of molecules of the peptide present in the sample. Such a signal - sometimes referred to herein as intensity signal - may be obtained, e.g., by measuring an intensity value of a specific physical or chemical property of the polypeptide. Indirect measuring includes measuring of a signal obtained from a secondary component, i.e. a component not being the polypeptide itself, or a biological read out system, e.g., measurable cellular responses, ligands, labels, or enzymatic reaction products.

In accordance with the present invention, determining the amount of the polypeptide, i.e. the biomarker as referred to herein can be achieved by all known means for determining the amount of a polypeptide or peptide in a sample. Said means comprise immunoassay devices and methods which may utilize labelled molecules in various sandwich, competition, or other assay formats. Said assays will develop a signal which is indicative of the presence or absence of the polypeptide. Moreover, the signal strength can, preferably, be correlated directly or indirectly (e.g. reverse-proportional) to the amount of polypeptide present in a sample. Further suitable methods comprise measuring a physical or chemical property specific for the polypeptide such as its precise molecular mass or NMR spectrum. Said methods comprise, preferably, biosensors, optical devices coupled to immunoassays, biochips, analytical devices such as mass-spectrometers, NMR-analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

It is also envisaged that the determining of the amount of a polypeptide comprises the steps of (a) contacting a cell capable of eliciting a cellular response the intensity of which is indicative of the amount of the polypeptide with the polypeptide for an adequate period of time, and (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the polypeptide.

Also preferably, determining the amount of the polypeptide comprises the step of measuring a specific intensity signal obtainable from a natriuretic polypeptide, such as NT-proBNP, or a pulmonary surfactant protein, such as proSP-B or C-fragment proSP-B, in the sample.

As described above, such a signal may be the signal intensity observed at an m/z variable specific for the polypeptide observed in mass spectra or a NMR spectrum specific for the polypeptide.

Further, determining the amount of a polypeptide, preferably, comprises the steps of (a) contacting the polypeptide or peptide with a specific ligand, (b) (optionally) removing non-bound ligand, and (c) measuring the amount of bound ligand. The bound ligand will generate an intensity signal. Binding according to the methods of the present invention includes both covalent and non-covalent binding. A ligand according to the methods of the present invention can be any compound, e.g., a peptide, polypeptide, nucleic acid, or small molecule, binding to the polypeptides described herein. Preferred ligands include antibodies, nucleic acids, peptides or polypeptides such as receptors for the polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also envisages the use of humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the polypeptide as referred to herein. Specific binding according to the present invention means that the ligand or agent should not bind substantially to ("cross-react" with) another peptide, polypeptide or substance present in the sample to be analysed. Preferably, the specifically bound polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative.

Suitable methods are described in the following. First, binding of a ligand may be measured directly, e.g. by NMR, mass spectrometry or surface plasmon resonance. Second, if the ligand also serves as a substrate of an enzymatic activity of the polypeptide of interest, an enzymatic reaction product may be measured (e.g., the amount of a protease can be measured by determining the amount of cleaved substrate, e.g. on a Western Blot). Alternatively, the ligand may exhibit enzymatic properties itself and the ligand/polypeptide complex or the ligand which was bound by the polypeptide, respectively, may be contacted with a suitable substrate allowing detection by the generation of an intensity signal. For measurement of enzymatic reaction products, preferably the amount of substrate is saturating. The substrate may also be labelled with a detectable label prior to the reaction. Preferably, the sample is contacted with the substrate for an adequate period of time. An adequate period of time refers to the time necessary for a detectable, preferably measurable, amount of product to be produced. Instead of measuring the amount of the product, the time necessary for appearance of a given (e.g., detectable) amount of product can be measured. Third, the ligand may be coupled covalently or non-covalently to a label allowing detection and measurement of the ligand. Labelling may be done by direct or indirect methods. Direct labelling involves coupling of the label directly (covalently or non-covalently) to the ligand. Indirect labelling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a coloured reaction product, fluorescence or chemoluminescence, which can be measured according to methods known in the art (e.g., using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g., Alexa 568). Further fluorescent labels are available, e.g., from Molecular Probes (Oregon). Also the use of quantum dots as fluorescent labels is contemplated. Typical radioactive labels include ³⁵5, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

Furthermore, determining the amount of a polypeptide preferably comprises (a) contacting a solid support comprising a ligand for the polypeptide as specified elsewhere herein with a sample comprising the polypeptide and (b) measuring the amount of the polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose stripes, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan JP, Sklar LA. (2002). Suspension array technology: evolution of the flat-array paradigm. Trends Biotechnol. 20(1):9-12). In such suspension arrays, the carrier, e.g. a microbead or microsphere, is present in suspension. The array consists of different microbeads or microspheres, possibly labelled, carrying different ligands. Methods of producing such arrays, for example based on solid-phase chemistry and photo-labile protective groups, are generally known (see, e.g., US 5,744,305).

The term "amount" as used herein encompasses the absolute amount of the polypeptides referred to herein, the relative amount or concentration of the polypeptides referred to herein as well as any value or parameter which correlates thereto. Such values or parameters comprise intensity signal values from all specific physical or chemical properties obtained from the polypeptide referred to herein by direct measurements, e.g., intensity values in mass spectra or NMR spectra. Moreover, encompassed are all values or parameters which are obtained by indirect measurements specified elsewhere in this description, e.g., expression levels determined from biological read out systems in response to the polypeptides referred to herein or intensity signals obtained from specifically bound ligands. It is to be understood that values correlating to the aforementioned amounts or parameters can also be obtained by all standard mathematical operations.

The term "natriuretic peptide" as used herein comprises Atrial Natriuretic Peptide (ANP)-type and Brain Natriuretic Peptide (BNP)-type peptides and variants thereof having the same diagnostic potential (see e.g. Bonow, 1996, Circulation 93: 1946-1950). ANP-type peptides comprise pre-proANP, proANP, NT-proANP, and ANP. BNP-type peptides comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renal. The in-vivo half-life of NT-proBNP is 120 min longer than that of BNP, which is 20 min (Smith 2000, J Endocrinol. 167: 239-46.). Pre-analytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measurement of the active or the inactive forms of the natriuretic peptide can be advantageous.

The preferred natriuretic peptides according to the present invention are NT-proBNP and variants thereof. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow, loc. cit. Preferably, human NT-proBNP as used herein is human NT-proBNP as disclosed in EP 0 648 228 B1.

The NT-proBNP referred to in accordance with the present invention further encompasses allelic and other variants of said specific sequence for human NT-proBNP discussed above. Specifically, envisaged are variant polypeptides which are on the amino acid level at least 60 % identical, more preferably at least 70 %, at least 80 %, at least 90 %, at least 95 %, at least 96%, at least 97%, at least 98%, or at least 99 % identical, to human NT-proBNP, preferably to the entire length of human NT-proBNP. The degree of identity between two amino acid sequences, in principle, can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Invest 59:177-181), Yeo et al. (Yeo 2003, Clinica Chimica Acta 338:107-115). Variants also include post-translational modified peptides such as glycosylated or myristylated peptides. Further, a variant in accordance with the present invention is also a peptide or polypeptide which has been modified after collection of the sample, for example by covalent or non-covalent attachment of a label, particularly a radioactive or fluorescent label, to the peptide. As set forth above, NT-proBNP and variants thereof can be used to diagnose heart failure and to grade the severity of that heart failure; see, e.g., WO2004/077056. Preferably, the amount of NT-proBNP in the sample of the heart failure patient is determined in the methods of the invention using Roche's electrochemiluminescence ELISA sandwich test Elecsys proBNP II STAT (Short Turn Around Time) assay described, e.g., in Example 1.

Pulmonary surfactant proteins are proteins which are under physiological conditions pivotally found in the pulmonary surfactant of a subject. Synthesised by Alveolar Type II cells, pulmonary surfactant phospholipids are stored in distinctive vesicles known as lamellar bodies. In response to a variety of stimuli, in particular physical distortion of the type II cells, the contents of the lamellar bodies are released into the hypophase, where they hydrate to form a 3-D lattice structure known as tubular myelin. The tubular myelin in turn supplies the monomolecular layer at the gas/liquid interface that possesses the biophysical activity. The components of the monomolecular layer have a defined life and are constantly replaced. The disaturated phospholipids are credited with reducing surface tension to the very low values thought to occur at low lung volumes, while cholesterol, the second most abundant pulmonary surfactant lipid, is thought to affect the rate of adsorption and the fluidity of newly released material. To date, four proteins, SP-A, -B, -C and -D have been shown to be uniquely associated with mammalian pulmonary surfactant. There is a general consensus that the extremely hydrophobic proteins SP-B and SP-C are functional components of the monomolecular layer, with SP-B being essential for the ability of surfactant to reduce surface tension, whereas the more hydrophilic protein, SP-A and SP-D, primarily mediate host-defence funtions.

Preferably, the term "pulmonary surfactant protein" as used herein refers to SP-B, more preferably to proSP-B and/or the C-fragment pro-SP-B which corresponds to amino acid residues 280 to 381 of SP-B as shown, for instance, in the amino acid sequence of accession number P07988.3. Synthesized in pulmonary Alveolar Epithelial Type II cells as a 381 amino acid propeptide, proSP-B undergoes sequential proteolytic cleavages from both the N-terminal and C-terminal ends and glycosylation to yield a 79 amino acid mature SP-B peptide. The term "proSP-B" as used herein means the hydrophilic 381 amino acid propeptide, whereas the term "SP-B" refers to the hydrophobic 79 amino acid mature SP-B peptide. Said terms, preferably, encompass the human proteins as well as variants thereof, preferably, allelic variants or species specific homologs, paralogs or orthologs. The human surfactant proteins are well characterized in the prior art and disclosed, e.g., in Hawgood, 1989, Am J Physiol -Lung Cellular and Molecular Physiology, Vol. 257, Issue 2:13-L22 (for all surfactant proteins), Takahashi 2006, Curr Pharm Des, 12(5):589-598 (for SP-A and SP-D), and Kurutz 2002, Biochemistry, 41(30):9627-9636, Guttentag 1998, Am J Physiol -Lung Cellular and Molecular Physiology, Vol. 275, Issue 3:L559-L566 (for SP-B). Specifically, envisaged are also variants of pulmonary surfactant proteins which are on the amino acid level at least 60 % identical, more preferably at least 70 %, at least 80 %, at least 90 %, at least 95 %, at least 96%, at least 97%, at least 98%, or at least 99 % identical, to the human pulmonary surfactant proteins, preferably to SP-B, more preferably to proSP-B and/or the C-fragment proSP-B. It is particularly preferred that the sequence identity is over the entire length of said proteins or fragments. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of a human pulmonary surfactant protein as long as the said polypeptides have pulmonary surfactant protein properties. Pulmonary surfactant protein properties as referred to herein are immunological and/or biological properties. Preferably, the pulmonary surfactant protein variants have immunological properties (i.e. epitope composition) comparable to those of the pulmonary surfactant proteins specifically referred to herein. Thus, the variants shall be recognizable by the aforementioned means or ligands used for determination of the pulmonary surfactant protein. Variants also include post-translational modified pulmonary surfactant proteins such as glycosylated proteins. Upon physical distortion or damage of the pulmonary Alveolar Epithelial Type II cells, hydrophilic pro-SP-B is released into the circulation. Such physical distortion or damage can occur under pathophysiological conditions, e.g. in heart failure (see, e.g., WO2004/077056), upon infection, upon artificial respiration or under toxic conditions (e.g., upon smoking). Preferably, the amount of proSP-B (and/or C-fragment proSP-B) in the methods of the invention is determined in the samples of heart failure patients by using the ELECSYS assay described, e.g., in Example 1.

The term "sample" refers to a sample of a body fluid, to a sample of separated cells or to a sample from a tissue or an organ. Samples of body fluids can be obtained by well-known techniques and include, preferably, samples of blood, plasma, serum or urine. Tissue or organ samples may be obtained from any tissue or organ by, e.g., biopsy. Separated cells may be obtained from the body fluids or the tissues or organs by separating techniques such as centrifugation or cell sorting (FACS). Preferably, the sample used in the methods of the invention is a blood sample, serum sample or plasma sample. Even more preferred, the sample is a serum sample.

"Comparing" as used herein encompasses comparing the amount or ratio of the polypeptides referred to herein which are comprised by the sample to be analysed with an amount of the said polypeptides in a suitable reference sample as specified elsewhere herein in this description. It is to be understood that comparing as used herein refers to a comparison of corresponding parameters or values, e.g., an absolute amount of a biomarker as referred to herein is compared to an absolute reference amount of said biomarker; a concentration of a biomarker as referred to herein is compared to a reference concentration of said biomarker; an intensity signal obtained from a biomarker as referred to herein in a test sample is compared to the same type of intensity signal of said biomarker in a reference sample; or a ratio of two biomarkers as referred to herein is compared to a reference ratio of said two biomarkers. The comparison referred to in the methods of the present invention may be carried out manually or computer assisted. For a computer assisted comparison, the value of the determined amount or ratio may be compared to values corresponding to suitable references which are stored in a database by a computer program. The computer program may further evaluate the result of the comparison by means of an expert system. Accordingly, a differential diagnosis for the diseases referred to herein may be automatically provided in a suitable output format.

Based on the comparison of the determined amount of a biomarker as referred to herein and the reference amount of said biomarker, or by the comparison of the determined ratio of two biomarkers as referred to herein to the reference ratio of said biomarkers, it shall be possible to assess whether the subject suffering from heart failure exhibits forward heart failure and/or backward heart failure of the left ventricle. Therefore, said reference amount or reference ratio is to be chosen so that either a difference or an identity in the compared amounts or ratios allows identifying those test subjects which belong into the group of subjects which have forward heart failure and/or backward heart failure of the left ventricle, or have not. The method allows either excluding (rule-out) or identifying (rule-in) a subject who has forward heart failure and/or backward heart failure of the left ventricle. Differences in the amounts or ratios, i.e. increases or decreases, as used herein, preferably, are differences which are statistically significant, as explained elsewhere herein.

The term "reference amount" or "reference level" or "reference value" as used herein refers to an amount of the biomarkers specified in this specification, preferably NT-proBNP or proSP-B or C-fragment proSP-B, which allows assessing which of the aforementioned diseases or disorders, i.e. forward heart failure and/or backward heart failure of the left ventricle, is the consequence of heart failure in a patient, by comparison the amounts of NT-proBNP or proSP-B or C-fragment proSP-B in a sample of said patient to a reference amount of the respective biomarker. A suitable reference amount may be determined from a reference sample to be analyzed together, i.e. simultaneously or subsequently, with the test sample.

Reference amounts can, in principle, be calculated for a cohort of subjects as specified herein based on the average or mean values for a given biomarker by applying standard methods of statistics. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity versus specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the methods of the present invention, i.e. a threshold which allows to discriminate between subjects who have forward heart failure and/or backward heart failure of the left ventricle or those who have not forward heart failure and/or backward heart failure of the left ventricle can be generated, preferably, by establishing a ROC for said cohort as described above and deriving a threshold amount there from. Dependent on a desired sensitivity and specificity for a diagnostic method, the ROC plot allows deriving suitable thresholds. It will be understood that an optimal sensitivity is desired for excluding forward heart failure and/or backward heart failure of the left ventricle (i.e. a rule out) whereas an optimal specificity is envisaged for a subject to be assessed to have forward heart failure and/or backward heart failure of the left ventricle (i.e. a rule in). Moreover, it is preferred that the amounts determined in the methods of the present invention are compared to more than one reference amounts, e.g. a reference amount for ruling in forward heart failure and/or backward heart failure of the left ventricle and a reference amount for ruling out forward heart failure and/or backward heart failure of the left ventricle.

Preferably, the reference amount as used herein is derived from a sample from a subject known to have forward heart failure, backward heart failure of the left ventricle or forward heart failure and backward heart failure of the left ventricle. The reference amount can also be derived, e.g., from one or more subjects of a relevant cohort known to have forward heart failure and/or backward heart failure of the left ventricle. Accordingly, by relevant cohort is meant a cohort characterized by one or more features which are also characteristic of the heart failure patient to be diagnosed by the methods of the invention. These features include, but are not limited to, age, gender, ethnicity, smoker/non-smoker status, or pulmonary health status. This reference amount level may be a discrete figure or may be a range of figures. Evidently, the reference level or amount may vary between individual classes of natriuretic peptides or surfactant molecules. For example, the normal level of SP-A may differ from the normal level of SP-B.

The reference amount applicable for an individual subject may vary depending on various physiological parameters such as age, gender, or subpopulation. Thus, a suitable reference amount may be determined by the methods of the present invention from a reference sample to be analysed together, i.e. simultaneously or subsequently, with the test sample. Moreover, a threshold amount can be preferably used as a reference amount. An amount of the polypeptides which is above the threshold amount or equal or below the threshold amount will be indicative for either forward heart failure and/or backward heart failure of the left ventricle as the cause of the heart failure. It is to be understood that the aforementioned amounts may vary due to statistics and errors of measurement.

In a further preferred embodiment of the present invention, the method further comprises the steps of determining the amount of proSP-B (or C-fragment proSP-B) and NT-proBNP in a sample from the heart failure patient, and determining or calculating the ratio of the amount of proSP-B (or C-fragment proSP-B) and the amount of NT-proBNP. Preferably, the calculated ratio is the ratio of proSP-B (or C-fragment proSP-B) to NT-proBNP. However, it is also envisaged that the calculated ratio is the ratio of NT-proBNP to proSP-B (or C-fragment proSP-B). The determination of both markers is advantageous, since the ratio of the amounts of both biomarkers allows for a particularly reliable diagnosis of forward heart failure and/or backward heart failure of the left ventricle in subjects with heart failure, as demonstrated in the following Examples.

In another preferred embodiment of the method of the invention, the amount of NT-proBNP in the sample from the reference heart failure patient is essentially the same as the amount of NT-proBNP in the patient to be tested. Preferred amounts of NT-proBNP in the sample from the reference heart failure patients are indicated elsewhere herein and in the following examples, in particular, in Table 2 of Example 3. The term "essentially identical" or "essentially the same" as used herein means that the amount of NT-proBNP in the sample from the reference heart failure patient may differ from the amount of NT-proBNP in the patient to be tested by plus/minus 10%, plus/minus 9%, plus/minus 8%, plus/minus 7%, plus/minus 6%, plus/minus 5%, plus/minus 4%, plus/minus 3%, plus/minus 2%, or plus/minus 1%. Preferably, the reference heart failure patient and the patient to be tested in the method of the invention fall into the same tertile group.

The following applies as diagnostic algorithm if the determined or calculated ratio is the ratio of proSP-B to NT-proBNP. As set forth elsewhere herein, the determined or calculated ratio in the method of the invention is preferably the ratio of proSP-B to NT-proBNP, wherein,
(i) a ratio of proSP-B to NT-proBNP in the test subject which is essentially identical as compared to the reference ratio from a sample of a reference heart failure patient suffering from forward heart failure, or which is lower than this reference ratio, is indicative for forward heart failure,
(ii) a ratio of proSP-B to NT-proBNP in the test subject which is essentially identical as compared to the reference ratio from a sample of a reference heart failure patient suffering from backward heart failure of the left ventricle, or which is higher than this reference ratio is indicative for backward heart failure of the left ventricle, and/or
(iii) a ratio of proSP-B to NT-proBNP in the test subject which is essentially identical as compared to the reference ratio from a sample of a reference heart failure patient suffering from forward heart failure and backward heart failure of the left ventricle is indicative for forward heart failure and backward heart failure of the left ventricle.

It is preferred that:
(i) the reference ratio of proSP-B to NT-proBNP for forward heart failure corresponds to about the 25^{th} percentile value of the ratio of proSP-B to NT-proBNP of Table 2. Preferably, said reference ratio is about 0.030, 0.035 or 0.040 for Tertile 1, about 0.015, 0.020 or 0.025 for Tertile 2 and about 0.003, 0.005 or 0.010 for Tertile 3. A ratio of proSP-B to NT-proBNP in the test subject which is essentially identical as compared to this reference ratio from a sample of a reference heart failure patient suffering from forward heart failure, or which is lower than this reference ratio, is indicative of forward heart failure.
(ii) the reference ratio of proSP-B to NT-proBNP for backward heart failure of the left ventricle corresponds to about the 75^{th} percentile value of the ratio of proSP-B to NT-proBNP of Table 2. Preferably, said reference ratio is about 0.180, 0.200 or 0.230 for Tertile 1, about 0.050, 0.060 or 0.080 for Tertile 2 and about 0.020, 0.030 or 0.040 for Tertile 3. A ratio of proSP-B to NT-proBNP in the test subject which is essentially identical as compared to this reference ratio from a sample of a reference heart failure patient suffering from backward heart failure of the left ventricle, or which is higher than this reference ratio is indicative of backward heart failure of the left ventricle.
(iii) the reference ratio of proSP-B to NT-proBNP for forward heart failure and backward heart failure of the left ventricle is preferably between about the 25^{th} and 75^{th} percentile value of the ratio of proSP-B to NT-proBNP of Table 2, or between about the 25^{th} and 75^{th} percentile value of the ratio of proSP-B to NT-proBNP as indicated in (i) or (ii) above. A test sample with a calculated ratio of proSP-B to NT-proBNP below the corresponding median value indicated in Table 2 (e.g., about 0.080 for Tertile 1, about 0.030 for Tertile 2 and about 0.010 for Tertile 3) is a sign for a predominance of forward heart failure in the heart failure patient, whereas a ratio above the median value is a sign for a predominance of backward failure of the left ventricle.

Further preferred reference ratios can be derived from the following Examples. It is evident to those skilled in the art that test samples of heart failure patients which show a ratio of proSP-B to NT-proBNP essentially identical or close to the 25^{th} or 75^{th} percentile value mentioned above are verified by a second method set forth elsewhere herein, thereby confirming the presence of forward heart failure and/or backward heart failure of the left ventricle.

The term "about" as used herein means plus/minus 30%, plus/minus 20%, plus/minus 10%, plus/minus 5%, plus/minus 4%, plus/minus 3%, plus/minus, 2% or plus/minus 1% from the specific value referred to.

The term "heart failure" is well known in the art. As used herein, the term, preferably, relates to an impaired systolic and/or diastolic function of the heart being accompanied by overt signs of heart failure. Preferably, heart failure referred to herein is chronic heart failure. More preferably, it is acute heart failure. The term "acute heart failure", preferably, refers to a worsening of cardiac function within a maximum of 2 weeks with or, in particular, without pre-existing chronic heart failure.

Heart failure (HF) can be classified into various degrees of severity. According to the NY-HA (New York Heart Association) classification, heart failure patients are classified as belonging to NYHA classes I, II, III and IV. A patient having heart failure has already experienced structural and functional changes to his pericardium, myocardium, coronary circulation or cardiac valves. He will not be able to fully restore his health, and is in need of a therapeutic treatment. Patients of NYHA Class I have no obvious symptoms of cardiovascular disease but already have objective evidence of functional impairment. Patients of NYHA class II have slight limitation of physical activity. Patients of NYHA class III show a marked limitation of physical activity. Patients of NYHA class IV are unable to carry out any physical activity without discomfort. They show symptoms of cardiac insufficiency at rest.

This functional classification is supplemented by the more recent classification by the American College of Cardiology and the American Heart Association (see J. Am. Coll. Cardiol. 2001; 38; 2101-2113, updated in 2005, see J. Am. Coll. Cardiol. 2005;46;e1-e82). Four stages A, B, C and D are defined. Stages A and B are not HF but are considered to help identify patients early before developing "truly" HF. Stages A and B patients are best defined as those with risk factors for the development of HF. For example, patients with coronary artery disease, hypertension, or diabetes mellitus who do not yet demonstrate impaired left ventricular (LV) function, hypertrophy, or geometric chamber distortion would be considered stage A, whereas patients who are asymptomatic but demonstrate LV hypertrophy and/or impaired LV function would be designated as stage B. Stage C then denotes patients with current or past symptoms of HF associated with underlying structural heart disease (the bulk of patients with HF), and stage D designates patients with truly refractory HF.

As used herein, the term "heart failure", preferably, refers to stages C and D of the ACC/AHA classification referred to above. In these stages, the subject shows typical symptoms of heart failure. Accordingly, a subject who suffers from heart failure, suffers from heart failure stage C or D according to the ACC/AHA classification. Alternatively, the term "heart failure" is classified as NYHA III or IV according to the NYHA classification. Even more preferred, the heart failure as used herein is decompensated heart failure. "Decompensated heart failure" as used herein is characterised by signs of congestion and/or hypoperfusion; signs of congestion include dyspnoe on exertion, proxysmal, nocturnal at rest or even orthopnoe, cough and wheezing, signs of hypoperfusion include confusion, altered mental status, dizziness, presyncope or syncope associated with cold extremities, narrow pulse pressure or pulsus alterans. In acute decompensation of chronic heart failure, such symptoms worsen. Decompensated heart failure results frequently from systolic chronic heart failure. Characteristic NT-proBNP levels for patients having systolic chronic heart failure correspond to about the 25^{th} percentile of decompensated heart failure. Accordingly, it is preferred that the heart failure patients to be tested in the methods of the invention exhibit an amount of NT-proBNP of higher than about 1700 ng/ml, or 1800 ng/ml or 1900 ng/ml or 2000 ng/ml in a sample, preferably a blood, serum or plasma sample. Even more preferred, said amount is higher than about 1900 ng/ml. Amounts below this value can be observed in diastolic heart failure and in heart failure without prior chronic heart failure.

Heart failure can also be classified as follows: acute versus chronic heart failure, systolic versus diastolic heart failure, high output versus low output heart failure, right sided versus left sided heart failure or forward versus backward heart failure, as briefly delineated below.

The prototype of acute heart failure is the patient who is entirely well but who suddenly develops a large myocardial infarction or rupture of a cardiac valve. Chronic heart failure is typically observed in patients with dilated cardiomyopathy or multivalvular heart disease that develops or progresses slowly. Acute heart failure is usually largely systolic and the sudden reduction in cardiac output often results in systemic hypotension without peripheral edema. In chronic heart failure, arterial pressure tends to be well maintained until very late in the course, but there is often accumulation of peripheral edema.

The systolic versus diastolic heart failure - classification relates to whether the principal abnormality is the inability to contract normally and expel sufficient blood (systolic failure) or to relax and fill normally (diastolic failure). The major clinical manifestations of systolic failure relate to an inadequate cardiac output with weakness, fatigue, reduced exercise tolerance and other symptoms of hypoperfusion, while in diastolic failure they relate principally to an elevation of filling pressures. In many patients, particularly those who have both ventricular hypertrophy and dilatation, abnormalities of contraction and relaxation coexist. Diastolic heart failure may be caused by increased resistance to ventricular inflow and reduced ventricular diastolic capacity (constrictive pericarditis and restrictive, hypertensive, and hypertrophic cardiomyopathy), impaired ventricular relaxation (acute myocardial ischemia, hypertrophic cardiomyopathy), and myocardial fibrosis and infiltration (dilated, chronic ischemic, and restrictive cardiomyopathy).

Low output heart failure occurs secondary to ischemic heart disease, hypertension, dilated cardiomyopathy, and valvular and pericardial disease. High output heart failure occurs in hyperthyroidism, anemia, pregnancy, arteriovenous fistulas, beriberi, liver diseases, sepsis and Paget's disease. In clinical practice, however, low output and high output heart failure cannot always be readily distinguished.

Patients in whom the left ventricle is mechanically overloaded (e.g., aortic stenosis) or weakened (e.g., post myocardial infarction) develop dyspnea and orthopnea as a result of pulmonary congestion, a condition referred to as left sided heart failure. In contrast, when the underlying abnormality affects the right ventricle primarily (e.g., pulmonic stenosis or pulmonary hypertension), symptoms resulting from pulmonary congestion such as orthopnea and paroxysmal nocturnal dyspnea are less common, and edema, congestive hepatomegaly, and systemic venous distention, i.e., clinical manifestations of right sided heart failure, are more prominent. However, when heart failure has existed for months or years, biventricular failure usually results. For example, patients with long standing aortic valve disease or systemic hypertension may have ankle edema, congestive hepatomegaly, and systemic venous distention late in the course of their disease, even though the abnormal hemodynamic burden initially was placed on the left ventricle.

In the case of forward heart failure, the clinical manifestations of heart failure result directly from an inadequate discharge of blood into the arterial system. Accordingly, salt and water retention is a consequence of diminished renal perfusion and excessive proximal tubular sodium reabsorption and of excessive distal tubular reabsorption through activation of the renin-angiotensin-aldosterone system. Accordingly, the term "forward heart failure" as used herein relates to the inability of the heart to pump blood at a sufficient rate to meet the oxygen demands of the body at rest or at exercise, i.e. the problem is primarily to supply adequate arterial perfusion in front of the heart preload. Forward heart failure is generally associated with low left ventricular output and peripheral ischemia. Forward heart failure can be diagnosed, e.g., by measuring the arterial blood pressure. Further diagnostic methods such as echocardiography (preferably in addition to Doppler data), radionuclide ventriculography (multiple-gated acquisition scanning), angiography (catheterization) or electrocardiogram (EKG or ECG) are well known in the art, as described, for example, in Hess O.M., Carroll J.D., Clinical assessment of heart failure, chapter 23, Braunwald's Heart Disease, 9th edition, page 561 ff.

The concept of backward heart failure contends that in heart failure, the right or left ventricle fails to discharge its contents or fails to fill normally. As a consequence, the pressures in the atrium and venous system behind the failing ventricle rise, and retention of sodium and water occurs as a consequence of the elevation of systemic venous and capillary pressures and the resultant transudation of fluids into the interstitial space. Accordingly, the term "backward heart failure" as used herein means the ability of the heart to pump blood at a sufficient rate only when heart filling pressures are abnormally high, i.e. the problem is primarily increased venous back pressure behind the heart afterload. The term "backward heart failure of the left ventricle" as used herein denotes a failure of proper function of the left ventricle. Backward failure of the left ventricle causes congestion of the pulmonary vasculature so that the symptoms are predominantly respiratory in nature. Backward failure of the right ventricle leads to congestion of systemic capillaries. Backward heart failure can be diagnosed, e.g., by methods specified elsewhere herein. Normal values for pulmonary arterial systolic pressure derived, e.g., from echochardiographic measurement are less than about 30 mmHG, less than about 25 mmHG, less than about 20 mmHG or less than about 15 mmHG.

The invention further pertains to a method for differentiating in a heart failure patient between (i) forward heart failure, (ii) backward heart failure of the left ventricle and (iii) forward heart failure and backward heart failure of the left ventricle, the method comprising the steps of:
a) determining in a sample of a heart failure patient the amount of proSP-B;
b) comparing the amount of proSP-B to at least one reference amount of proSP-B, thereby differentiating between (i) forward heart failure, (ii) backward heart failure of the left ventricle and (iii) forward heart failure and backward heart failure of the left ventricle in said heart failure patient.

In a preferred embodiment of this method of the invention, the at least one reference amount is derived (i) from a sample of a reference heart failure patient suffering from forward heart failure, (ii) from a sample of a reference heart failure patient suffering from backward heart failure of the left ventricle and/or (iii) from a sample of a reference heart failure patient suffering from forward heart failure and backward heart failure of the left ventricle.

In another preferred embodiment of this method of the invention, the amount of NT-proBNP in the sample from the reference heart failure patient is essentially the same as the amount of NT-proBNP in the patient to be tested.

In a still further preferred embodiment of this method of the invention,
(i) an amount of proSP-B in the test subject which is essentially identical as compared to the reference amount from a sample of a reference heart failure patient suffering from forward heart failure, or which is lower than said reference amount is indicative for forward heart failure,
(ii) an amount of proSP-B in the test subject which is essentially identical as compared to the reference amount from a sample of a reference heart failure patient suffering from backward heart failure of the left ventricle, or, which is higher than said reference amount is indicative for backward heart failure, and/or
(iii) an amount of proSP-B in the test subject which is essentially identical as compared to the reference amount from a sample of a reference heart failure patient suffering from forward heart failure and backward heart failure of the left ventricle is indicative for forward heart failure and backward heart failure of the left ventricle.

It is preferred that:
(i) the reference amount of proSP-B for forward heart failure corresponds to about the 25^{th} percentile value of proSP-B of Table 2. Accordingly, said reference amount is preferably about 30, 40 or 65 ng/ml for Tertile 1, about 70 or 79 ng/ml for Tertile 2 and about 90 or 102 ng/ml for Tertile 3. An amount of proSP-B in the test subject which is essentially identical to this reference amount, or which is lower than said reference amount, is indicative of forward heart failure.
(ii) the reference amount of proSP-B for backward heart failure of the left ventricle corresponds to about the 75^{th} percentile value of proSP-B of Table 2. Accordingly, said reference amount is preferably about 163, 170 or 180 ng/ml for Tertile 1, about 218, 230 or 270 ng/ml for Tertile 2 and about 213, 220 or 270 ng/ml for Tertile 3. An amount of proSP-B in the test subject which is essentially identical to this reference amount or which is higher than said reference amount is indicative of backward heart failure of the left ventricle.
(iii) the reference amount of proSP-B for forward heart failure and backward heart failure of the left ventricle is between about the 25^{th} and 75^{th} percentile value of proSP-B of Table 2 or between about the 25^{th} and 75^{th} percentile value of proSP-B indicated in (i) or (ii) above. A test sample with an amount of proSP-B below the corresponding median value indicated in Table 2 (e.g. about 105 ng/ml for Tertile 1, about 127 ng/ml for Tertile 2, and about 157 ng/ml for Tertile 3) is a sign for a predominance of forward heart failure in the heart failure patient, whereas an amount above the median value is a sign for a predominance of backward failure of the left ventricle.

Further preferred reference amounts can be derived from the following Examples. It is evident to those skilled in the art that test samples of heart failure patients which show an amount of proSP-B essentially identical or close to the 25^{th} or 75^{th} percentile value mentioned above are verified by a second method set forth elsewhere herein, thereby confirming the presence of forward heart failure and/or backward heart failure of the left ventricle.

Encompassed by the invention is also a method for differentiating in a heart failure patient between (i) forward heart failure and (ii) backward heart failure of the left ventricle exhibiting NT-proBNP amounts corresponding to or above the 25^{th} percentile of decompensated heart failure, the method comprising the steps of:
a) determining in a sample of a heart failure patient the amount of proSP-B;
b) comparing the amount of proSP-B to at least one reference amount of proSP-B, thereby differentiating between (i) forward heart failure and (ii) backward heart failure of the left ventricle in said heart failure patient.

Preferably, the NT-proBNP amounts corresponding to the 25^{th} percentile of decompensated heart failure are about 1700 pg/ml, or 1800 pg/ml, or 1900 pg/ml, or 2000 pg/ml in a sample, preferably a blood, serum or plasma sample of a heart failure patient. Even more preferred, said amount is or is higher than about 1900 pg/ml.

The invention relates also to a method for monitoring in a heart failure patient with a significant increase or decrease of the amount of NT-proBNP (i) forward heart failure, (ii) backward heart failure of the left ventricle and (iii) forward heart failure and backward heart failure of the left ventricle, the method comprising the steps of:
a) determining the amount of proSP-B in a first sample of a heart failure patient at a first timepoint;
b) determining the amount of proSP-B in a second sample of said heart failure patient at a second timepoint, said second sample being obtained after said first sample, and, optionally;
c) comparing the amount of proSP-B determined in the first sample with the amount of proSP-B determined in the second sample,
wherein a stable amount of proSP-B or a decrease of the amount of proSP-B in the second sample relative to the first sample is indicative for (i) forward heart failure or (iii) forward heart failure and backward heart failure of the left ventricle, and an increase of the amount of proSP-B in the second sample is indicative for (ii) backward heart failure of the left ventricle.

In a preferred embodiment, the heart failure patient with a significant increase or decrease of the amount of NT-proBNP has exhibited an increase of at least 5%, 10%, 15%, 20%, 25% or even more in a sample, preferably a blood, serum or plasma sample. Even more preferred, said increase or decrease is at least 10%.

In a further preferred embodiment, the heart failure patient with a significant increase or decrease of the amount of NT-proBNP has exhibited said increase or decrease within a period of at least about 3 hours, 4 hours, 5 hours, 6 hours, 12 hours, 24 hours, 2 days, 3 days, 4 days, 5 days, 6 days, 7 days, 10 days, two weeks, three weeks, four weeks, six weeks, 2 months, 3 months or even longer, prior to carrying out said method. It is further preferred that said first and second samples are obtained at intervals of 1, 2, 3, or 4 weeks, or 2, 3, or 4 months.

The method for monitoring (i) forward heart failure, (ii) backward heart failure of the left ventricle and (iii) forward heart failure and backward heart failure of the left ventricle in a heart failure patient of the invention can be used in order to follow blood levels of proSP-B (and/or the C-fragment proSP-B) in a heart failure patient, for example, during therapy. For instance, worsening of cardiac function in a heart failure patient has been found to be associated with an increase of proSP-B as a sign of backward heart failure (TP 2) which improved as cardiac function improved (TP 3), as shown in Example 4.

Preferably, the heart failure is advanced heart failure, more preferably stage C, even more preferably stage D heart failure.

The basis therapy for heart failure has been described elsewhere herein. The appropriate specific therapy of backward failure of the left ventricle is to deprive the patient of fluid which includes, e.g., the reduction of the intravasal volume of the heart failure patient, whereas forward heart failure requires measures which help to improve the cardiac output, such as volume substitution, inotropes and/or ASSIST devices. Accordingly, in a preferred embodiment of the method of the invention, (i) forward heart failure can be treated by volume substitution, inotropes and/or ASSIST devices, (ii) backward heart failure of the left ventricle can be treated by reduction of the intravasal volume of the heart failure patient, and (iii) forward and backward heart failure of the left ventricle can be treated, e.g, by angiotensin converting enzyme (ACE) inhibitors, angiotensin II receptor blockers (ARBs), beta blockers and/or diuretics.

While NT-proBNP and proSP-B have been linked to heart failure and NYHA status in previous studies, so far no association has been provided between said biomarkers and consequences of heart failure. The present invention for the first time provides for a method which allows for the distinction of forward heart failure and backward heart failure of the left ventricle in a heart failure patient, by analyzing the amounts of the biomarkers NT-proBNP and proSP-B in a sample from a heart failure patient. Since (i) forward heart failure, (ii) backward heart failure of the left ventricle and (iii) forward heart failure and backward heart failure of the left ventricle have to be treated differently, the method of the invention further provides for treatment guidance for the treating physician(s). Accordingly, the term "treatment guidance" as used herein means that the inventive method can serve as a guideline for an appropriate therapy, thereby allowing a more precise treatment regimen, in comparison to those described in the prior art.

In principle, the basic treatment of heart failure maybe divided logically into three components: (1) removal of the precipitating cause, (2) correction of the underlying cause, and (3) control of heart failure symptoms. The control of heart failure symptoms, may, in turn, be divided into three categories: (i) reduction of cardiac workload, including both preload and afterload; (ii) control of excessive retention of salt and water; and (iii) enhancement of myocardial contractility. Treatment of heart failure is well described in the art; see, e.g., D.G. Mann, Clinical assessment of heart failure (HF), chapter 25, Braunwald's Heart Disease, 9th edition, page 611 ff. Clinical heart failure patients have been subdivided into four 'stages.' Stage A. patients are at high risk for developing clinical HF (i.e., those with hypertension, diabetes, dyslipidaemia, and so on), but without detectable structural heart disease. Stage B. patients have detectable structural heart disease (i.e. LVH, LV dysfunction), but no clinical signs or symptoms of HF. Stage C. patients have current or past clinical HF. Stage D. patients are patients with end-stage refractory HF, who are candidates for extraordinary forms of therapy or for compassionate end-of-life care. Stage A. and B. patients can be treated, e.g., by ACE inhibitors and/or ARBs. Stage C. patients can receive, in addition, to ACE inhibitors and/or ARBs, beta blockers and diuretics, selected patients may obtain biventricular pacing or implantable defibrillators. Stage D. patients can be treated, e.g., by chronic inotropes, permanent mechanical support or heart transplantation.

Though a simple rule for the treatment of all patients with heart failure cannot be formulated because of varied etiologies, hemodynamic features, clinical manifestations, and severity of heart failure, the methods of the present invention can help to improve the treatment of heart failure, by distinguishing forward heart failure and/or backward heart failure of the left ventricle. Based on the results of this differential diagnosis, a specific therapy can be applied which is adapted to the treatment of forward heart failure or backward heart failure of the left ventricle. More specifically, forward heart failure is generally associated with low left ventricular output and peripheral ischemia. In this case, volume substitution can be indicated in order to avoid cardio-renal syndrome. Furthermore, inotropes such as doputamine can be useful in this scenario. ASSIST devices can also be helpful to support output in cases that do not respond to pharmacotherapy; see, e.g., Naka Y. and E.A. Rose, Clinical assessment of heart failure (HF), chapter 28, Braunwald's Heart Disease, 9th edition, page 685 ff. For example, ASSIST devices can be temporarily used in chronic heart failure patients to bridge the time to transplantation or can be permanently implanted into the apex of the left ventricle in order to prolong life expectancy of the respective patient. In contrast, in case of pulmonary congestion as is the case in backward heart failure, reduction of intravasal volume is the therapy of choice. This includes the use of loop diuretics or vasodilators such as nitrates. Where applicable, this specific treatment for forward heart failure or backward heart failure of the left ventricle can be applied, in addition, to the basic heart failure therapy described above, for example, in patients having both forward heart failure and backward heart failure of the left ventricle. In such cases, it is decisive for the treatment which of forward heart failure or backward heart failure of the left ventricle is prevailing, as explained elsewhere herein.

Diagnostic tests currently utilised to diagnose heart failure comprise, e.g., echocardiography, radionuclide ventriculography (multiple-gated acquisition scanning), angiography (catheterization) or electrocardiogram (EKG or ECG). However, there can be either patient reluctance or physician reluctance to order such tests in the absence of one or more risk factor indicators (e.g., obesity or diabetes) or actual symptomatic evidence (e.g., shortness of breath). Since not all sufferers of heart failure exhibit one or more of the well known risk factors, this can often mean that heart failure is not detected until it has become severe and/or chronic, thereby leading to an increase in the incidence of mortality and a significant burden to the health system since late stage diagnosis usually involves more expensive and interventionist monitoring and treatment, as opposed to the simpler therapeutic measures and/or changes in lifestyle which may suffice if diagnosis occurred at an early stage. In light of this, dissection of different types of heart failure as enabled by the methods of the present invention has clear therapeutic consequences and advantageous adds to clinical signs and symptoms which might not be present at early stages. Moreover, it will allow to follow up the spontaneous course of heart failure and help to monitor effects of treatment.

In a preferred embodiment of the methods of the invention, the forward heart failure is characterized by the inability of the heart to pump blood at a sufficient rate to meet the oxygen demand of the body at rest or exercise.

In another preferred embodiment of the methods of the invention, the backward heart failure of the left ventricle is characterized by the ability of the heart to pump blood at a sufficient rate only when the heart filling pressures are abnormally high.

In a still preferred embodiment of the methods of the invention, the sample is a blood sample, plasma sample or serum sample.

The specification further describes the use of (i) proSP-B and NT-proBNP, (ii) a detection agent which specifically binds to proSP-B and/or (iii) a detection agent which specifically binds to NT-proBNP in a sample of a heart failure patient for differentiating in said heart failure patient forward heart failure and/or backward heart failure of the left ventricle. Such detection agents which specifically bind to proSP-B, C-fragment proSP-B or to NT-proBNP and appopriate qualitative and quantitative assays for the detection of said proteins have been described elsewhere herein. Preferably, antibodies specifically binding to proSP-B, C-fragment proSP-B or to NT-proBNP are used to this end in immunoassays.

The specification further describes the use of proSP-B or a detection agent which specifically binds to proSP-B in a sample of a heart failure patient for diagnosing backward heart failure of the left ventricle in said heart failure patient.

The definitions of the terms and the embodiments with respect to the methods of the invention apply *mutatis mutandis* to the uses described herein.

The specification also describes a device for differentiating in a heart failure patient forward heart failure and/or backward heart failure of the left ventricle, comprising:
a) an analysing unit for determining the amount of proSP-B and NT-proBNP in a sample of a heart failure patient;
b) an evaluation unit for comparing the determined amount with a suitable reference amount and for differentiating forward heart failure and/or backward heart failure of the left ventricle in said heart failure patient.

In addition, the specification describes a device for diagnosing in a heart failure patient backward heart failure of the left ventricle, comprising:
a) an analysing unit for determining the amount of proSP-B in a sample of a heart failure patient;
b) an evaluation unit for comparing the determined amount with a suitable reference amount and for diagnosing backward heart failure of the left ventricle in said heart failure patient.

The term "device" as used herein relates to a system of means comprising at least the aforementioned means operatively linked to each other as to allow the prediction. Preferred means for determining the amount of the said polypeptides and means for carrying out the comparison are disclosed above in connection with the methods of the invention. How to link the means in an operating manner will depend on the type of means included into the device. For example, where means for automatically determining the amount of the polypeptides or peptides are applied, the data obtained by said automatically operating means can be processed by, e.g., a computer program in order to diagnose or distinguish between the diseases referred to herein. Preferably, the means are comprised by a single device in such a case. Said device may accordingly include an analyzing unit for the measurement of the amount of the polypeptides or peptides in a sample and a computer unit for processing the resulting data for the differential diagnosis. Alternatively, where means such as test stripes are used for determining the amount of the polypeptides, the means for diagnosing may comprise control stripes or tables allocating the determined amount to an amount known to be accompanied with forward heart failure and/or backward heart failure of the left ventricle. The test stripes are, preferably, coupled to a ligand which specifically binds to the polypeptides as defined elsewhere in this specification. The strip or device, preferably, comprises means for detection of the binding of said (poly)peptides to the said ligand. Preferred means for detection are disclosed in connection with embodiments relating to the methods of the invention above. In such a case, the means are operatively linked in that the user of the system brings together the result of the determination of the amount and the diagnostic value thereof due to the instructions and interpretations given in a manual. The means may appear as separate devices and are, preferably, packaged together as a kit. The person skilled in the art will realize how to link the means without further inventive skills. Preferred devices are those which can be applied without the particular knowledge of a specialized clinician, e.g., test stripes or electronic devices which merely require loading with a sample. The results may be given as output of parametric diagnostic raw data, preferably, as absolute or relative amounts. It is to be understood that these data will need interpretation by the clinician. However, also envisaged are expert system devices wherein the output comprises processed diagnostic raw data the interpretation of which does not require a specialized clinician. Further preferred devices comprise the analyzing units/devices (e.g., biosensors, arrays, solid supports coupled to ligands specifically recognizing the polypeptides, Plasmon surface resonance devices, NMR spectrometers, mass- spectrometers etc.) or evaluation units/devices referred to above in accordance with the methods of the invention.

The specification also describes a kit for differentiating in a heart failure patient forward heart failure and/or backward heart failure of the left ventricle, comprising:
a) an analysing unit for determining the amount of proSP-B and NT-proBNP in a sample of a heart failure patient;
b) an evaluation unit for comparing the determined amount with a suitable reference amount and for differentiating forward heart failure and/or backward heart failure of the left ventricle in said heart failure patient.

Finally, the specification describes a kit for diagnosing in a heart failure patient backward heart failure of the left ventricle, comprising:
a) an analysing unit for determining the amount of proSP-B in a sample of a heart failure patient;
b) an evaluation unit for comparing the determined amount with a suitable reference amount and for diagnosing backward heart failure of the left ventricle in said heart failure patient.

The definitions of the terms and the embodiments with respect to the methods of the invention apply *mutatis mutandis* to the devices and kits described herein.

The term "kit" as used herein refers to a collection of the aforementioned means, preferably, provided in separately or within a single container. The container, also preferably, comprises instructions for carrying out the method of the present invention. The invention, thus, relates to a kit comprising a means or an agent for measuring a polypeptide referred to herein. Examples for such means or agents as well as methods for their use have been given in this specification. The kit, preferably, contains the aforementioned means or agents in a ready-to-use manner. Preferably, the kit may additionally comprise instructions, e.g., a user's manual for interpreting the results of any determination(s) with respect to the diagnoses provided by the methods of the present invention. Particularly, such manual may include information for allocating the amounts of the determined polypeptides to the kind of diagnosis. Details are to be found elsewhere in this specification. Additionally, such user's manual may provide instructions about correctly using the components of the kit for determining the amount(s) of the respective biomarker. A user's manual may be provided in paper or electronic form, e.g., stored on CD or CD ROM. The specification also describes the use of said kit in any of the methods according to the present invention.

### Figures:

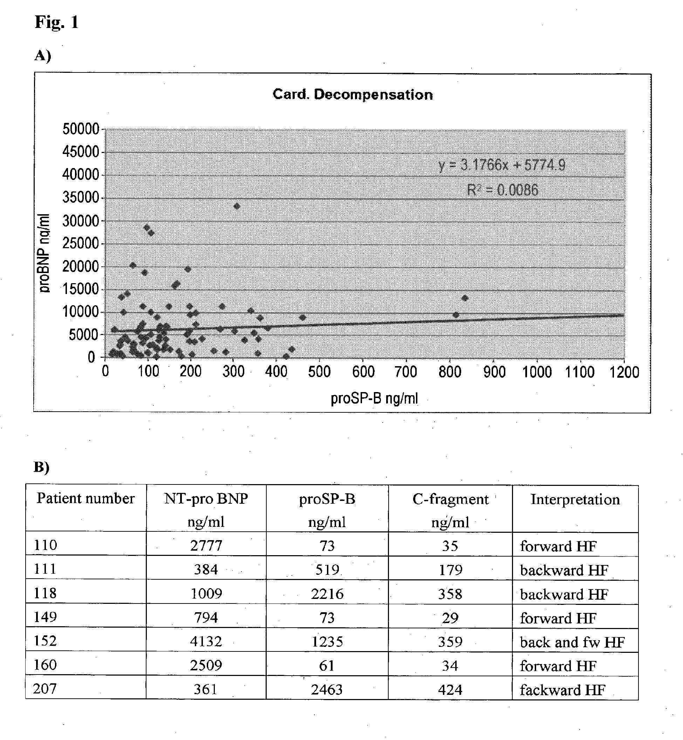

**Figure 1:** A) Correlation of NT-pro BNP to proSP-B. ProSP-B and NT-proBNP did not correlate confirming that NT-proBNP as a biomarker for cardiac function and proSP-B as a biomarker of pulmonary damage provide different information. B) Examples of specific patients further illustrate this finding.
**Figure 2****:** Correlation between proSP-B and C-fragment proSP-B concentrations. ProSP-B levels correlated quite well to C-fragment proSP-B levels, except in a few patients who had high proSP-B and C-fragment proSP-B concentrations.

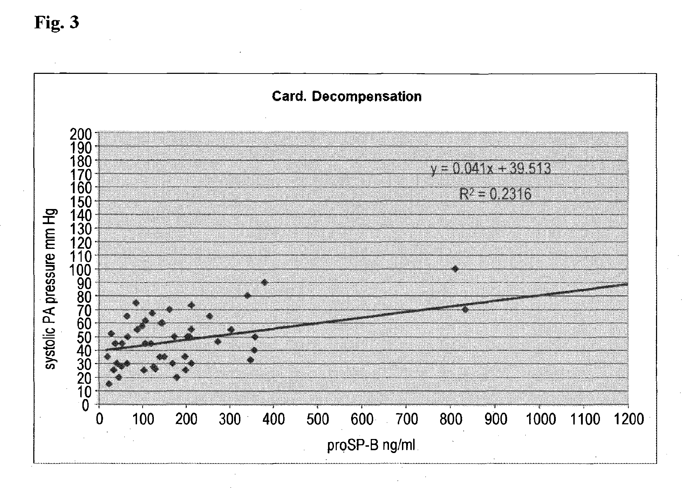

**Figure 3****:** Correlation between pro SP-B and systolic PA pressure. In patients with cardiac decompensation, pro SP-B concentrations tended to increase with systolic pulmonary artery pressure which is considered to be a sign of backward heart failure.

### Examples:

The invention will now be illustrated by the following examples which shall, however, not be construed as limiting the scope of the invention.

### Example 1:

NT-proBNP was determined in serum using Roche's electrochemiluminescence ELISA sandwich test Elecsys proBNP II STAT (Short Turn Around Time) assay. The test employs two monoclonal antibodies which recognize epitopes located in the N-terminal part (1-76) of proBNP (1-108).

ProSP-B and C-fragment proSP-B were tested in serum using a newly developed immunoassay according to the ELCSYS principle. The proSP-B test uses two monoclonal antibodies, one directed to the N-terminus and one directed to the C-terminus (C fragment proSP-B corresponding to amino acid residues 280 to 381) of the proSP-B molecule. The C-fragment proSP-B test thereby detects the different degradation forms of this molecule.

### Example 2:

A total of 74 patients with clinically stable coronary artery disease (CAD) confirmed by angiography were included into the study. All patients had an unimpaired left ventricular ejection fraction (LVEF) above 60 % as tested by echocardiography, a normal chest X-ray and they had no clinical symptoms; in addition, physical examination revealed no signs of heart failure such as rale, 3^{rd} or 4^{th} heart sound. In addition, they had no obvious concomitant diseases and, specifically, not of the lung. Moreover, the patients did not smoke. They also had no impaired kidney function, as assessed by creatinine levels within normal ranges.

Results are summarized in the following Table 1, indicating the median and, in addition, the 25^{th} and 75^{th} percentile in parenthesis.

**Table 1:**

| | NT-proBNP (pg/ml) | proSP-B (ng/ml) | C-fragment proSP-B (ng/ml) |
|---|---|---|---|
| Total group N = 74 | 144 (67/283) | 29 (23/52) | 60 (42/104) |
| Subgroup below median of NT-pro BNP n = 37 | 63 (35-96) | 27 (22/49) | 59 (42/91) |
| Subgroup above median of NT-pro BNP n = 37 | 287 (223-322) | 34 (25/54) | 76 (44/107) |

The data obtained for proSP-B and C-fragment proSP-B in patients with CAD are compatible to normal subjects who did not smoke, indicating that moderate cardiac dysfunction does not result in significant alveolar damage.

Triple blood drawings at a single timepoint and analysis of the proSP-B and C-fragment proSP-B showed that the variations in the results obtained were less than 5 %, indicating that changes above this limit had to be considered as a change in pulmonary damage in the individual patient.

### Example 3:

A further group of 92 patients with decompensated left sided heart failure were also tested. The included patients did not suffer from an independent lung disorder. They were admitted to the emergency room because of worsening of symptoms of heart failure within the past two weeks. All patients were on standard heart failure therapy which included beta blockers, ACE inhibitors and/or angiotensin receptor blockers and diuretics.

Results are summarized in the following Table 2, indicating the median and, in addition, the 25^{th} and 75^{th} percentile in parenthesis.

**Table 2:**

| | NT-proBNP | proSP-B | C-fragment proSP-B | proSP-B/ NT-proBNP | C-fragment proSP-B /NT-proBNP |
|---|---|---|---|---|---|
| | (pg/ml) | (ng/ml) | (ng/ml) | | |
| All patients | 4478 (1878-8908) | 127 (79-211) | 296 (157-483) | | |
| Tertile 1 | 1228 (810-1878) | 105 (65-163) | 246 (133-442) | 0.08 (0.04-0.18) | 0.16 (0.09-0.44) |
| Tertile 2 | 4478 (3719-5773) | 127 (79-218) | 311 (155-551) | 0.03 (0.02-0.05) | 0.07 (0.04-0.12) |
| Tertile 3 | 10739 (9152-14867) | 157 (102-213) | 350 (212-505) | 0.01 (0.01-0.02) | 0.03 (0.02-0.059 |

As can be seen from Figure 1, when NT-proBNP, as a marker of cardiac function, was correlated to proSP-B, as a marker of lung damage, both markers did not provide identical information.

When proSP-B was correlated to C-fragment proSP-B, both markers of lung damage provided largely identical information, indicating that the information provided by said markers is comparable, as can be derived from Figure 2.

When the pulmonary artery pressure which was assessed by echocardiography was correlated to proSP-B, increased proSP-B concentrations were related to increased pulmonary artery pressure, whereby the PA pressure represents a sign of (long standing) backward heart failure, see Figure 3. Accordingly, backward failure of the left ventricle has been found to be associated with an increased proSP-B level, most likely as a result of the congestion of the pulmonary vasculature which leads to leakage of the hydrophilic proSP-B from the Alveolar Type II cells into the circulation. In contrast, there is no or less congestion of the pulmonary vasculature reflected in non-elevated proSP-B levels, in patients suffering from backward heart failure of the right ventricle. Thus, increased proSP-B amount is a marker of backward failure of the left ventricle.

The determination of both markers NT-proBNP and proSP-B (or C-fragment proSP-B) is advantageous, since the ratio of the amounts of both biomarkers allows for a particularly reliable diagnosis of forward heart failure and/or backward heart failure of the left ventricle in subjects with heart failure. Thus, the combined assessment of NT-proBNP and proSP-B (or C-fragment proSP-B) allows a more definite dissection of patients suffering from heart failure, by differentiating between forward heart failure and backward heart failure (of the left ventricle). This is also shown in the individual examples in Figure 1B.

Patients characterised as forward heart failure had lower systemic blood pressure (91 plus/minus 8 mmHg), than patients with backward heart failure (112 plus/minus 12 mmHg). Patients with forward heart failure had also a lower LVEF than patients with backward heart failure of the left ventricle (2 % vs 40 %, respectively). In addition, they had very faint peripheral pulses and cold extremities; the latter features were not present in patients diagnosed with backward heart failure of the left ventricle.

### Example 4:

A total of 32 patients with clinically stable heart failure were also included into the study. All patients were on standard heart failure therapy which included beta blockers, ACE inhibitors, and/or angiotensin receptor blockers and diuretics. Patients were seen at 2 weeks interval, resulting in a total of 3 visits for each patient. During that period, treatment remained stable and clinical events did not occur. Kidney function was normal at entry into the study and remained so for the duration of the study.

Results are summarised below in Table 3, indicating the median and the 25^{th} and 75^{th} percentile in parenthesis.

**Table 3:**

| | NT-proBNP (pg/ml) | proSP-B (ng/ml) | C-fragment proSP-B (ng/ml) |
|---|---|---|---|
| Timepoint 1 (TP1) | 356 (184-820) | 53 (34-67) | 111 (67-153) |
| Timepoint 2 (TP2) | 424 (188-938) | 51 (34-96) | 107 (66-211) |
| Timepoint 3 (TP3) | 476 (219-719) | 51 (29-89) | 104 (64-209) |

In all patients, median values for NT-proBNP slightly increased and proSP-B remained unchanged, except for two patients. These cases are shown below in Table 4, with TP representing time point.

**Table 4:**

| **Patient 1** | | | |
|---|---|---|---|
| | NT-proBNP (pg/ml) | proSP-B (ng/ml) | C-fragment proSP-B (ng/ml) |
| TP 1 | 1008 | 62 | 116 |
| TP 2 | 1773 | 152 | 443 |
| TP 3 | 1319 | 68 | 128 |

| **Patient 2** | | | |
|---|---|---|---|
| | NT-proBNP (pg/ml) | proSP-B (ng/ml) | C-fragment proSP-B (ng/ml) |
| TP 1 | 1092 | 39 | 102 |
| TP 2 | 1497 | 39 | 88 |
| TP 3 | 2058 | 48 | 95 |

As can be seen in patient 1, worsening of cardiac function was associated with an increase of proSP-B as a sign of backward heart failure (TP 2) which improved as cardiac function improved (TP 3). This patient had also higher proSP-B concentrations at entry into the study, as compared to patient 2. Both had similar NT-pro BNP levels at study entry. In contrast thereto, patient 2 showed no evidence of backward heart failure and his amounts of proSP-B and C-fragment proSP-B remained stable, although NT-proBNP increased significantly, indicating forward heart failure.

### Example 5:

A total of 16 patients with different stages of aortic valve stenosis were tested before and 3 months after valve replacement.

Results are summarized in the following Table 5 indicating the median and the 25^{th} and 75^{th} percentile.

**Table 5:**

| | NT-proBNP (pg/ml) | proSP-B (ng/ml) | C-fragment proSP-B (ng/ml) |
|---|---|---|---|
| T0 (before replacement) | 1415 (476/3320) | 91 (58/149) | 196 (115/378) |
| T1 (3 months after replacement) | 461 (295/1015) | 60 (43/126) | 128 (97/258) |

As can be seen from the Table 5, median values for NT-proBNP decreased significantly after aortic valve replacement, indicating improvement of cardiac function. This was also the case for secondary pulmonary involvement. The value of combining NT-proBNP and proSP-B (or C-fragment pro SP-B) measurement is demonstrated for specific examples shown in Table 6.

**Table 6:**

| | NT-proBNP (pg/ml) | proSP-B (ng/ml) | C-fragment proSP-B (ng/ml) |
|---|---|---|---|
| **Case 30** | | | |
| T0 (before replacement) | 200 | 42 | 92 |
| T1 (3 months after replacement) | 211 | 50 | 98 |

| **Case 21** | | | |
|---|---|---|---|
| T0 (before replacement) | 731 | 30 | 90 |
| T1 (3 months after replacement) | 430 | 31 | 73 |

The two cases above showed moderate cardiac dysfunction and no significant pulmonary involvement. While case 30 showed no change after valve replacement, case 21 has improvement in forward heart failure without evidence of backward heart failure.

**Table 7:**

| | NT-proBNP (pg/ml) | proSP-B (ng/ml) | C-fragment proSP-B (ng/ml) |
|---|---|---|---|
| **Case 26** | | | |
| T0 (before replacement) | 10300 | 301 | 475 |
| T1 (3 months after | 779 | 122 | 277 |

| **Case 42** | | | |
|---|---|---|---|
| T0 (before replacement) | 6645 | 317 | 548 |
| T1 (3 months after replacement) | 325 | 37 | 75 |

| **Case 37** | | | |
|---|---|---|---|
| T0 (before replacement) | 4935 | 169 | 451 |
| T1 (3 months after replacement) | 222 | 148 | 416 |

| **Case 14** | | | |
|---|---|---|---|
| T0 (before replacement) | 2593 | 265 | 597 |
| T1 (3 months after replacement) | 2197 | 138 | 346 |

As can be seen from the individual cases in Table 7, aortic valve disease was associated with significant cardiac dysfunction and secondary pulmonary involvement in all patients. All but one (Case 14) had significant improvement in cardiac function. This was associated with normalisation of pulmonary involvement in one patient (case 42) and improvement in two patients (cases 26 and 37). Case 14 showed improvement of backward heart failure with no or little improvement of cardiac function and forward heart failure.

Thus, the individual cases show the clinical value of the combined measurement of NT-proBNP and proSP-B (or C-fragment proSP-B) in patient assessment with respect to the predominant form of heart failure, i.e. backward heart failure, forward heart failure or combined.

Valve replacement was associated with a significant improvement of cardiac output and, thus, of cardiac function as assessed by echocardiography (and decrease of NT-proBNP values). The decrease in the amount of proSP-B was associated with disappearance of rales in those patients who had such signs before valve replacement.

### Example 6:

As demonstrated in Examples 1 to 5, the combined determination of proSP-B and/or C-fragment proSP-B and NT-proBNP allows to estimate the predominant form of left heart failure. Backward heart failure which is associated with congestion of the lung can be treated by reduction of intravasal volume, e.g. the use of loop diuretics initially and thereafter careful balancing with alternative diuretics in combination with other heart failure drugs such as ACE inhibitors, ARBs, beta blockers and calcium antagonists. In contrast to cases of forward heart failure depending on the underlying mechanism, treatment by pacemakers, defibrillators, assist devices or drugs may be indicated (such as dobutamine). Thus, the combined measurement of proSP-B and NT-proBNP allows for a more sophisticated treatment of left heart failure.

## Claims

1. A method for differentiating in a heart failure patient between (i) forward heart failure, (ii) backward heart failure of the left ventricle and (iii) forward heart failure and backward heart failure of the left ventricle, the method comprising the steps of:
a) determining in a sample of a heart failure patient the ratio of the amount of proSP-B and the amount ofNT-proBNP; and
b) comparing the ratio of proSP-B and NT-proBNP determined in step a) to at least one reference ratio of proSP-B and NT-proBNP,
thereby differentiating between (i) forward heart failure, (ii) backward heart failure of the left ventricle and (iii) forward heart failure and backward heart failure of the left ventricle in said heart failure patient.

2. The method of claim 1, wherein the at least one reference ratio is derived (i) from a sample of a reference heart failure patient suffering from forward heart failure, (ii) from a sample of a reference heart failure patient suffering from backward heart failure of the left ventricle and/or (iii) from a sample of a reference heart failure patient suffering from forward heart failure and backward heart failure of the left ventricle.

3. The method of claim 1 or 2, wherein the determined ratio is the ratio of proSP-B to NT-proBNP , and wherein,
(i) a ratio of proSP-B to NT-proBNP in the test subject which is essentially identical as compared to the reference ratio from a sample of a reference heart failure patient suffering from forward heart failure, or, which is lower than said reference ratio, is indicative for forward heart failure,
(ii) a ratio of proSP-B to NT-proBNP in the test subject which is essentially identical as compared to the reference ratio from a sample of a reference heart failure patient suffering from backward heart failure of the left ventricle, or, which is higher than said reference ratio is indicative for backward heart failure, and/or
(iii) a ratio of proSP-B to NT-proBNP in the test subject which is essentially identical as compared to the reference ratio from a sample of a reference heart failure patient suffering from forward heart failure and backward heart failure of the left ventricle is indicative for forward heart failure and backward heart failure of the left ventricle.

4. A method for differentiating in a heart failure patient between (i) forward heart failure, (ii) backward heart failure of the left ventricle and (iii) forward heart failure and backward heart failure of the left ventricle, the method comprising the steps of:
a) determining in a sample of a heart failure patient the amount of proSP-B;
b) comparing the amount of proSP-B to at least one reference amount of proSP-B, thereby differentiating between (i) forward heart failure, (ii) backward heart failure of the left ventricle and (iii) forward heart failure and backward heart failure of the left ventricle in said heart failure patient.

5. A method for monitoring in a heart failure patient with a significant increase or decrease of the amount of NT-proBNP (i) forward heart failure, (ii) backward heart failure of the left ventricle and (iii) forward heart failure and backward heart failure of the left ventricle, the method comprising the steps of:
a) determining the amount of proSP-B in a first sample of a heart failure patient at a first timepoint;
b) determining the amount of proSP-B in a second sample of said heart failure patient at a second timepoint, said second sample being obtained after said first sample, and, optionally;
c) comparing the amount of proSP-B determined in the first sample with the amount of proSP-B determined in the second sample,
wherein a stable amount of proSP-B or a decrease of the amount of proSP-B in the second sample relative to the first sample is indicative for (i) forward heart failure or (iii) forward heart failure and backward heart failure of the left ventricle, and an increase of the amount of proSP-B in the second sample is indicative for (ii) backward heart failure of the left ventricle.

6. The method of claim 5, wherein the heart failure is advanced heart failure, preferably stage C, more preferably stage D heart failure.

7. The method of any of claims 1 to 6, wherein the forward heart failure is **characterized by** the inability of the heart to pump blood at a sufficient rate to meet the oxygen demand of the body at rest or exercise.

8. The method of any of claims 1 to 6, wherein the backward heart failure of the left ventricle is **characterized by** the ability of the heart to pump blood at a sufficient rate only when the heart filling pressures are abnormally high.

9. The method of any of claims 1 to 8, wherein the sample is a blood, serum or plasma sample.

## Patentansprüche

1. Verfahren zur Differenzierung in einem Patienten mit Herzinsuffizienz zwischen (i) Vorwärtsherzversagen, (ii) Rückwärtsherzversagen des linken Ventrikels und (iii) Vorwärtsherzversagen und Rückwärtsherzversagen des linken Ventrikels, wobei das Verfahren folgende Schritte umfasst:
a) Bestimmen des Verhältnisses der Menge von proSP-B und der Menge von NT-proBNP in einer Probe eines Herzinsuffizienz-Patienten; und
b) Vergleichen des in Schritt a) bestimmten Verhältnisses von proSP-B und NT-proBNP mit zumindest einem Referenzverhältnis von proSP-B und NT-proBNP, wodurch bei dem Herzinsuffizienz-Patienten zwischen (i) Vorwärtsherzversagen, (ii) Rückwärtsherzversagen des linken Ventrikels und (iii) Vorwärtsherzversagen und Rückwärtsherzversagen des linken Ventrikels differenziert werden kann.

2. Verfahren nach Anspruch 1, worin das zumindest eine Referenzverhältnis (i) von einer Probe eines Herzinsuffizienz-Referenzpatienten, der an Vorwärtsherzversagen leidet, (ii) von einer Probe eines Herzinsuffizienz-Referenzpatienten, der an Rückwärtsherzversagen des linken Ventrikels leidet, und/oder (iii) von einer Probe eines Herzinsuffizienz-Referenzpatienten, der an Vorwärtsherzversagen und Rückwärtsherzversagen des linken Ventrikels leidet, stammt.

3. Verfahren nach Anspruch 1 oder 2, worin das bestimmte Verhältnis das Verhältnis von proSP-B zu NT-proBNP ist, und worin
(i) ein Verhältnis von proSP-B zu NT-proBNP der Testperson, das im Vergleich zu dem Referenzverhältnis von einer Probe eines Herzinsuffizienz-Referenzpatienten, der an Vorwärtsherzversagen leidet, im Wesentlichen identisch ist, oder das niedriger als das Referenzverhältnis ist, auf Vorwärtsherzversagen hinweist,
(ii) ein Verhältnis von proSP-B zu NT-proBNP der Testperson, das im Vergleich zu dem Referenzverhältnis von einer Probe eines Herzinsuffizienz-Referenzpatienten, der an Rückwärtsherzversagen des linken Ventrikels leidet, im Wesentlichen identisch ist, oder das höher als das Referenzverhältnis ist, auf Rückwärtsherzversagen hinweist, und/oder
(iii) ein Verhältnis von proSP-B zu NT-proBNP der Testperson, das im Vergleich zu dem Referenzverhältnis von einer Probe eines Herzinsuffizienz-Referenzpatienten, der an Vorwärtsherzversagen und Rückwärtsherzversagen des linken Ventrikels leidet, im Wesentlichen identisch ist, auf Vorwärtsherzversagen und Rückwärtsherzversagen des linken Ventrikels hinweist.

4. Verfahren zur Differenzierung in einem Patienten mit Herzinsuffizienz zwischen (i) Vorwärtsherzversagen, (ii) Rückwärtsherzversagen des linken Ventrikels und (iii) Vorwärtsherzversagen und Rückwärtsherzversagen des linken Ventrikels, wobei das Verfahren folgende Schritte umfasst:
a) Bestimmen der Menge von proSP-B in einer Probe eines Herzinsuffizienz-Patienten;
b) Vergleichen der Menge von proSP-B mit zumindest einer Referenzmenge von proSP-B,
wodurch bei dem Herzinsuffizienz-Patienten zwischen (i) Vorwärtsherzversagen, (ii) Rückwärtsherzversagen des linken Ventrikels und (iii) Vorwärtsherzversagen und Rückwärtsherzversagen des linken Ventrikels in dem Herzinsuffizienz-Patienten differenziert werden kann.

5. Verfahren zur Überwachung in einem Herzinsuffizienz-Patienten mit signifikanter Zunahme oder Abnahme der Menge von NT-proBNP von (i) Vorwärtsherzversagen, (ii) Rückwärtsherzversagen des linken Ventrikels und (iii) Vorwärtsherzversagen und Rückwärtsherzversagen des linken Ventrikels, wobei das Verfahren die folgenden Schritte umfasst:
a) Bestimmen der Menge von proSP-B in einer ersten Probe eines Herzinsuffizienz-Patienten zu einem ersten Zeitpunkt;
b) Bestimmen der Menge von proSP-B in einer zweiten Probe des Herzinsuffizienz-Patienten zu einem zweiten Zeitpunkt, wobei die zweite Probe nach der ersten Probe entnommen wurde, und fakultativ
b) Vergleichen der Menge von proSP-B, die in der ersten Probe bestimmt wurde, mit der in der zweiten Probe bestimmten Menge von proSP-B,
worin eine stabile Menge von proSP-B oder eine Abnahme der Menge von proSP-B in der zweiten Probe im Verhältnis zu der ersten Probe auf (i) Vorwärtsherzversagen oder (iii) Vorwärtsherzversagen und Rückwärtsherzversagen des linken Ventrikels hinweist, und eine Zunahme der Menge von proSP-B in der zweiten Probe auf (ii) Rückwärtsherzversagen des linken Ventrikels hinweist.

6. Verfahren nach Anspruch 5, worin die Herzinsuffizienz fortgeschrittene Herzinsuffizienz ist, vorzugsweise Herzinsuffizienz in Stadium C, und insbesondere Herzinsuffizienz im Stadium D.

7. Verfahren nach einem der Ansprüche 1 bis 6, worin das Vorwärtsherzversagen **dadurch gekennzeichnet ist, dass** das Herz nicht in der Lage ist, Blut mit einer Rate zu pumpen, die zur Deckung des Sauerstoffbedarfs des Körpers in Ruhe oder bei Belastung ausreicht.

8. Verfahren nach einem der Ansprüche 1 bis 6, worin das Rückwärtsherzversagen des linken Ventrikels **dadurch gekennzeichnet ist, dass** das Herz nur dann in der Lage ist, Blut mit einer ausreichenden Rate zu pumpen, wenn der Fülldruck des Herzens abnorm hoch ist.

9. Verfahren nach einem der Ansprüche 1 bis 8, worin die Probe eine Blut-, Serum- oder Plasmaprobe ist.

## Revendications

1. Procédé pour la différenciation, chez un patient présentant une insuffisance cardiaque, entre (i) une insuffisance cardiaque antérograde, (ii) une insuffisance cardiaque rétrograde du ventricule gauche et (iii) une insuffisance cardiaque antérograde et une insuffisance cardiaque rétrograde du ventricule gauche, le procédé comprenant les étapes dans lesquelles :
a) on détermine, dans un échantillon d'un patient présentant une insuffisance cardiaque, le rapport entre la quantité de proSP-B et la quantité de NT-proBNP ; et
b) on compare le rapport entre le proSP-B et le NT-proBNP que l'on détermine à l'étape a) à au moins un rapport de référence entre le proSP-B et le NT-proBNP ;
pour ainsi opérer une différenciation entre (i) une insuffisance cardiaque antérograde, (ii) une insuffisance cardiaque rétrograde du ventricule gauche et (iii) une insuffisance cardiaque antérograde et une insuffisance cardiaque rétrograde du ventricule gauche, chez ledit patient présentant une insuffisance cardiaque.

2. Procédé selon la revendication 1, dans lequel on dérive ledit au moins un rapport de référence (i) d'un échantillon d'un patient de référence présentant une insuffisance cardiaque souffrant d'une insuffisance cardiaque antérograde ; (ii) d'un échantillon d'un patient de référence présentant une insuffisance cardiaque souffrant d'une insuffisance cardiaque rétrograde du ventricule gauche ; et/ou (iii) d'un échantillon d'un patient de référence présentant une insuffisance cardiaque souffrant d'une insuffisance cardiaque antérograde et d'une insuffisance cardiaque rétrograde du ventricule gauche.

3. Procédé selon la revendication 1 ou 2, dans lequel le rapport déterminé représente le rapport entre le proSP-B et le NT-proBNP, et dans lequel
(i) un rapport entre le proSP-B et le NT-proBNP dans le sujet soumis au test qui est essentiellement identique au rapport de référence émanant d'un échantillon d'un patient de référence présentant une défaillance cardiaque souffrant d'une défaillance cardiaque antérograde ou qui est inférieur audit rapport de référence, est révélateur d'une insuffisance cardiaque antérograde ;
(ii) un rapport entre le proSP-B et le NT-proBNP dans le sujet soumis au test qui est essentiellement identique au rapport de référence émanant d'un échantillon d'un patient de référence présentant une défaillance cardiaque souffrant d'une défaillance cardiaque rétrograde du ventricule gauche ou qui est supérieur audit rapport de référence, est révélateur d'une insuffisance cardiaque rétrograde ; et/ou
(iii) un rapport entre le proSP-B et le NT-proBNP dans le sujet soumis au test qui est essentiellement identique au rapport de référence émanant d'un échantillon d'un patient de référence présentant une défaillance cardiaque souffrant d'une insuffisance cardiaque antérograde et d'une insuffisance cardiaque rétrograde du ventricule gauche, est révélateur d'une insuffisance cardiaque antérograde et d'une insuffisance cardiaque rétrograde du ventricule gauche.

4. Procédé pour la différenciation, chez un patient présentant une insuffisance cardiaque, entre (i) une insuffisance cardiaque antérograde, (ii) une insuffisance cardiaque rétrograde du ventricule gauche et (iii) une insuffisance cardiaque antérograde et une insuffisance cardiaque rétrograde du ventricule gauche, le procédé comprenant les étapes dans lesquelles :
a) on détermine, dans un échantillon d'un patient présentant une insuffisance cardiaque, la quantité de proSP-B ;
b) on compare la quantité de proSP-B à au moins une quantité de référence de proSP-B, pour ainsi opérer une différenciation entre (i) une insuffisance cardiaque antérograde, (ii) une insuffisance cardiaque rétrograde du ventricule gauche et (iii) une insuffisance cardiaque antérograde et une insuffisance cardiaque rétrograde du ventricule gauche, chez ledit patient présentant une insuffisance cardiaque.

5. Procédé pour la surveillance, chez un patient présentant une insuffisance cardiaque qui manifeste une augmentation ou une diminution significative de la quantité de NT-proBNP, (i) d'une insuffisance cardiaque antérograde, (ii) d'une insuffisance cardiaque rétrograde du ventricule gauche et (iii) d'une insuffisance cardiaque antérograde et d'une insuffisance cardiaque rétrograde du ventricule gauche, le procédé comprenant les étapes dans lesquelles :
a) on détermine la quantité de proSP-B dans un premier échantillon d'un patient présentant une insuffisance cardiaque, à un premier moment ;
b) on détermine la quantité de proSP-B dans un deuxième échantillon dudit patient présentant une insuffisance cardiaque, à un deuxième moment, ledit deuxième échantillon étant obtenu après ledit premier échantillon ; et, de manière facultative
c) on compare la quantité de proSP-B déterminée dans le premier échantillon à la quantité de proSP-B déterminée dans le deuxième échantillon ;
dans lequel une quantité stable de proSP-B ou une diminution de la quantité de proSP-B dans le deuxième échantillon par rapport à la quantité du premier échantillon est révélatrice (i) d'une insuffisance cardiaque antérograde ou (iii) d'une insuffisance cardiaque antérograde et une insuffisance cardiaque rétrograde du ventricule gauche, et une augmentation de la quantité de proSP-B dans le deuxième échantillon est révélatrice (ii) d'une insuffisance cardiaque rétrograde du ventricule gauche.

6. Procédé selon la revendication 5, dans lequel l'insuffisance cardiaque représente une insuffisance cardiaque avancée, de préférence de stade C, de manière plus préférée une insuffisance cardiaque de stade D.

7. Procédé selon l'une quelconque des revendications 1 à 6, dans lequel l'insuffisance cardiaque antérograde est **caractérisée par** l'incapacité du coeur à pomper du sang à une fréquence suffisante pour répondre à la demande du corps en oxygène au repos ou en activité.

8. Procédé selon l'une quelconque des revendications 1 à 6, dans lequel l'insuffisance cardiaque rétrograde du ventricule gauche est **caractérisée par** la capacité du coeur à pomper du sang à une fréquence suffisante uniquement lorsque les pressions de remplissage cardiaque sont anormalement élevées.

9. Procédé selon l'une quelconque des revendications 1 à 8, dans lequel l'échantillon est un échantillon de sang, de sérum ou de plasma.
